(19) Europäisches Patentamt
European Patent Office
Office européen des brevets

(11) EP 4 043 480 A1

(12) # EUROPEAN PATENT APPLICATION
published in accordance with Art. 153(4) EPC

(43) Date of publication:
**17.08.2022 Bulletin 2022/33**

(21) Application number: **19948373.6**

(22) Date of filing: **12.10.2019**

(51) International Patent Classification (IPC):
***C07K 14/00*** *(2006.01)* ***C07K 1/06*** *(2006.01)*
***C07K 1/04*** *(2006.01)* ***A61K 38/16*** *(2006.01)*
***A61P 3/04*** *(2006.01)* ***A61P 3/10*** *(2006.01)*

(86) International application number:
**PCT/CN2019/110848**

(87) International publication number:
**WO 2021/068251 (15.04.2021 Gazette 2021/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.10.2019 CN 201910952193**

(71) Applicants:
- **Sinopep-Allsino Biopharmaceutical Co., Ltd.**
  **Lianyungang, Jiangsu 222000 (CN)**
- **Hangzhou Sinopep-Allsino Pharmaceutical Technology**
  **Development Co., Ltd**
  **Hangzhou, Zhejiang 311100 (CN)**

(72) Inventors:
- **ZHAO, Chengqing**
  **Lianyungang, Jiangsu 222000 (CN)**
- **GU, Haitao**
  **Lianyungang, Jiangsu 222000 (CN)**
- **SHI, Guoqiang**
  **Lianyungang, Jiangsu 222000 (CN)**
- **JIANG, Jianjun**
  **Lianyungang, Jiangsu 222000 (CN)**
- **WANG, Caidian**
  **Lianyungang, Jiangsu 222000 (CN)**

(74) Representative: **Bandpay & Greuter**
**30, rue Notre-Dame des Victoires**
**75002 Paris (FR)**

(54) **GIP AND GLP-1 DUAL AGONIST POLYPEPTIDE COMPOUND, PHARMACEUTICALLY ACCEPTABLE SALT OF SAME, AND USES THEREOF**

(57) A GIP and GLP-1 dual agonist polypeptide compound, related to the technical field of polypeptide medicinal chemistry. The amino acid sequence of the GIP and GLP-1 dual agonist polypeptide compound is: $YXaa^1EGTFTSDYSIXaa^2LDKIAQXaa^3AFVQWLIAGGPSSGAPPPS$; moreover, the amino acid at the C-terminus of the polypeptide compound is amidated to serve as a C-terminus primary amide. A preparation method for the polypeptide compound, a pharmaceutically acceptable salt, a pharmaceutical composition, and a pharmaceutical preparation. The polypeptide compound provides at the same time sugar-reducing and weight-loss effects and is applicable as an active ingredient in preparing a medicament for treating or preventing diabetes or a weight-loss medicament.

EP 4 043 480 A1

SEQUENCE LISTING

<110> SINOPEP-ALLSINO BIOPHARMACEUTICAL CO., LTD.
HANGZHOU SINOPEP-ALLSINO PHARMACEUTICAL TECHNOLOGY DEVELOPMENT CO., LTD.

<120> GIP AND GLP-1 DUAL AGONIST POLYPEPTIDE COMPOUND, PHARMACEUTICALLY ACCEPTABLE SALT AND APPLICATION THEREOF

<160> 1

<170> SIPOSequenceListing 1.0

<210> 1
<211> 39
<212> PRT
<213> Artificial Sequence

<400> 1

```
Tyr Val Glu Gly Thr Phe Thr Ser Asp Tyr Ser Ile Val Leu Asp Lys
1               5                   10                  15
Ile Ala Gln Lys Ala Phe Val Gln Trp Leu Ile Ala Gly Gly Pro Ser
            20                  25                  30
Ser Gly Ala Pro Pro Pro Ser
        35
```

## Description

### TECHNICAL FIELD

The present invention relates to the field of polypeptide compounds, and particularly relates to a double-intestinal insulinotropic peptide analogue compound, a polypeptide compound. The polypeptide compound can activate receptors of human glucose-dependent insulinotropic polypeptide (GIP) and glucagon-like peptide-1 (GLP-1) and can be used for treating type 2 diabetes. The present invention further relates to a pharmaceutically acceptable salt of the polypeptide compound, a polypeptide pharmaceutical composition, a method and an application thereof.

### BACKGROUND

The pathogenesis of metabolic syndrome is abnormal metabolism of various substances such as protein, fat and carbohydrate. Overnutrition and reduced physical activity can lead to obesity and obesity-related diseases such as diabetes. In recent years, the incidence of type 2 diabetes and dyslipidemia is on the rise; type 2 diabetes is the most common form of diabetes, accounting for about 90% of all diabetes. Type 2 diabetes is characterized by high blood glucose levels caused by insulin resistance. The current standard of care for type 2 diabetes includes diet and exercise and the availability of oral and injectable hypoglycemic agents. Nevertheless, many patients with type 2 diabetes remain inadequately controlled.

Glucose-dependent insulinotropic peptide (GIP) is a 42-amino acid gastrointestinal regulatory peptide that stimulates insulin secretion from pancreatic beta cells and protects pancreatic β-cells in the presence of glucose, playing a physiological role in glucose homeostasis. GLP-1 is a 37-amino acid peptide that stimulates insulin secretion, protects pancreatic beta cells, and inhibits glucagon secretion, gastric emptying, and food intake, resulting in weight loss. GIP and GLP-1 are known as incretin; Incretin receptor signaling transmission has a physiologically relevant role to maintain glucose homeostasis. In normal physiology, GIP and GLP-1 are secreted from the gut after a meal, and these incretins enhance physiological responses to foods, including satiety, insulin secretion, and nutrient processing. The incretin response is impaired in patients with type 2 diabetes. Currently commercially available incretin analogs or dipeptidyl peptidase IV (DPP-IV) inhibitors utilize a single mechanism of action for glycemic control; if a compound for type 2 diabetes with a dual mechanism of action is used, a polypeptide compound with excellent hypoglycemic activity and weight loss effect can be obtained.

It has been found that the dosage of GLP-1 analogs is limited by side effects such as nausea and vomiting, and therefore administration of a drug often cannot achieve the full efficacy in glycemic control and weight loss. GIP alone has very modest glucose-lowering capacity in patients with type 2 diabetes. Both native GIP and GLP-1 are rapidly inactivated by the ubiquitous protease DPP IV, therefore, they can only be used for short-term metabolic control. DPP IV belongs to the exopeptidase class of proteolytic enzymes; the introduction of unnatural amino acids into the sequence can increase the proteolytic stability of any given peptide, although unnatural amino acids help stabilize the peptide against DPP IV proteolysis and other forms of degradation.

As a part of the present invention, the unnatural amino acids positively affect the balance of agonist activity between GIP and GLP-1. For example, fatty acids can improve the pharmacokinetics of peptides by extending half-life through their albumin-binding motifs. While fatty acids can improve the half-life of peptides, the applicant has discovered that, as a part of the present invention, the length, composition and location of the fatty acid chains and the linkers between the peptides and the fatty acid chains may have unexpected effect on the balance of activity of GIP and GLP-1 agonists while extending the half-life of the peptide. Some GIP analogs exhibit GIP and GLP-1 activity, as disclosed in the patents such as WO2013/164483, WO2014/192284 and WO2011/119657, etc.

### SUMMARY

In view of the drawbacks of the prior art, an object of the present invention is to provide a GIP and GLP-1 dual agonist polypeptide compound which utilizes a dual-action mechanism and has blood glucose reducing activity and weight-losing effect.

Another object of the present invention further is to provide a method for preparing the GIP and GLP-1 dual agonist polypeptide compound.

Still another object of the present invention is to provide a pharmaceutically acceptable salt of the GIP and GLP-1 dual agonist polypeptide compound.

Still another object of the present invention is to provide a pharmaceutical composition of the GIP and GLP-1 dual agonist polypeptide compound.

Still another object of the present invention is to provide a medicament of the GIP and GLP-1 dual agonist polypeptide compound.

Still another object of the present invention is to provide the GIP and GLP-1 dual agonist polypeptide compound, a pharmaceutically acceptable salt thereof, a pharmaceutical composition thereof and an application of a medicament.

The following technical solutions are used to achieve the objects.

The present invention discloses a GIP and GLP-1 dual agonist polypeptide compound. The GIP and GLP-1 dual agonist polypeptide compound is characterized in that the amino acid sequence of the GIP and GLP-1 dual agonist polypeptide compound is $YXaa^1EGTFTSDYSIXaa^2LDKIAQXaa^3AFVQWLIAGGPSSGAPPPS$, and the C-terminal amino acid of the polypeptide compound is amidated as C-terminal primary amide; wherein:

$Xaa^1$ is: V, Aib , A,

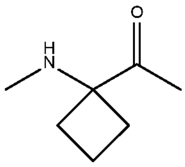 , 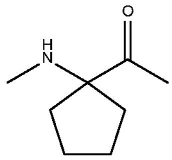 , 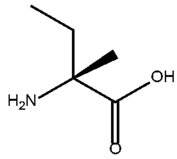 or 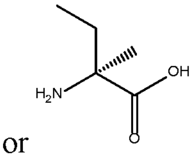 ;

$Xaa^2$ is: V, Aib, A,

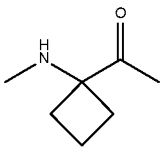 , 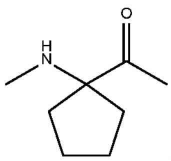 , 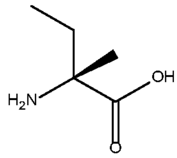 or 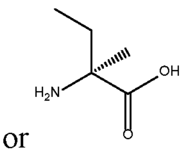 ;

$Xaa^1$ and $Xaa^2$ are the same or different;

$Xaa^3$ is:

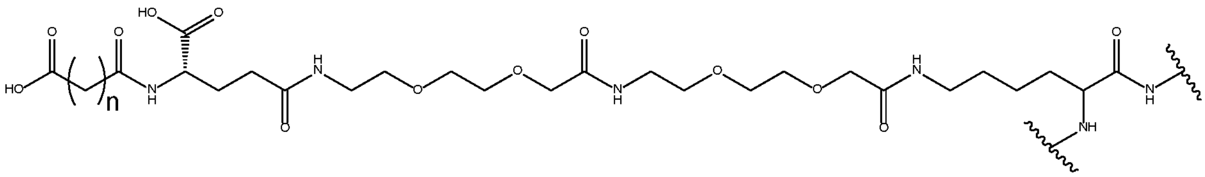

or:

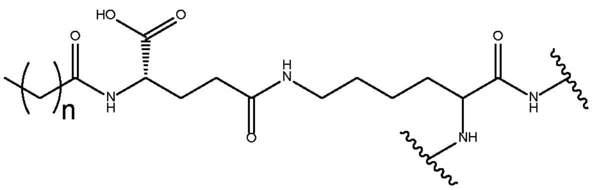

n is a natural number ranging from 5 to 25.

Further, n is a natural number 16 or 18.

Further, the amino acid sequence of the polypeptide compound is preferably shown as follows:

(1) SEQ ID NO: 1

YVEGTFTSDYSIVLDKIAQK(HOOC-$(CH_2)_{16}$-CO-γ-Glu-AEEA-AEEA)AFVQWLIAGGPSSGAPPPS-$NH_2$

(2) SEQ ID NO: 2

YXEGTFTSDYSIXLDKIAQK(HOOC-$(CH_2)_{16}$-CO-γ-Glu-AEEA-AEEA)AFVQWLIAGGPSSGAPPPS-$NH_2$

(3) SEQ ID NO: 3

$YX_1EGTFTSDYSIX_1LDKIAQK$(HOOC-$(CH_2)_{16}$-CO-γ-Glu-AEEA-AEEA)AFVQWLIAGGPSSGAPPPS-$NH_2$

(4) SEQ ID NO: 4

YAEGTFTSDYSIALDKIAQK(HOOC-$(CH_2)_{16}$-CO-γ-Glu-AEEA-AEEA)AFVQWLIAGGPSSGAPPPS-$NH_2$

(5) SEQ ID NO: 5

YAEGTFTSDYSIAibLDKIAQK(HOOC-$(CH_2)_{16}$-CO-γ-Glu-AEEA-AEEA)AFVQWLIAGGPSSGAPPPS-$NH_2$

(6) SEQ ID NO: 6

YAibEGTFTSDYSIALDKIAQK(HOOC-$(CH_2)_{16}$-CO-γ-Glu-AEEA-AEEA)AFVQWLIAGGPSSGAPPPS-$NH_2$

(7) SEQ ID NO: 7

$YXEGTFTSDYSIX_2LDKIAQK$(HOOC-$(CH_2)_{16}$-CO-γ-Glu-AEEA-AEEA)AFVQWLIAGGPSSGAPPPS-$NH_2$

(8) SEQ ID NO: 8

$YXEGTFTSDYSIX_2LDKIAQK$(HOOC-$(CH_2)_{16}$-CO-γ-Glu-AEEA-AEEA)AFVQWLIAGGPSSGAPPPS-$NH_2$

(9) SEQ ID NO: 9

$YX_1EGTFTSDYSIX_2LDKIAQK$(HOOC-$(CH_2)_{16}$-CO-γ-Glu-AEEA-AEEA)AFVQWLIAGGPSSGAPPPS-$NH_2$

(10) SEQ ID NO: 10

YX$_2$EGTFTSDYSIX$_1$LDKIAQK(HOOC-$(CH_2)_{16}$-CO-γ-Glu-AEEA-AEEA)AFVQWLIAGGPSSGAPPPS-$NH_2$

(11) SEQ ID NO: 11
YAibEGTFTSDYSIAibLDKIAQK(γ-Glu-Palmitoyl)AFVQWLIAGGPSSGAPPPS-$NH_2$
(12) SEQ ID NO: 12
YVEGTFTSDYSIVLDKIAQK(γ-Glu-Palmitoyl)AFVQWLIAGGPSSGAPPPS-$NH_2$
(13) SEQ ID NO: 13
YXEGTFTSDYSIXLDKIAQK(γ-Glu-Palmitoyl)AFVQWLIAGGPSSGAPPPS-$NH_2$
(14) SEQ ID NO: 14
YX$_1$EGTFTSDYSIX$_1$LDKIAQK(γ-Glu-Palmitoyl)AFVQWLIAGGPSSGAPPPS-$NH_2$
(15) SEQ ID NO: 15
YAEGTFTSDYSIALDKIAQK(γ-Glu-Palmitoyl)AFVQWLIAGGPSSGAPPPS-$NH_2$
(16) SEQ ID NO: 16
YAEGTFTSDYSIAibLDKIAQK(γ-Glu-Palmitoyl)AFVQWLIAGGPSSGAPPPS-$NH_2$
(17) SEQ ID NO: 17
YAibEGTFTSDYSIALDKIAQK(γ-Glu-Palmitoyl)AFVQWLIAGGPSSGAPPPS-$NH_2$
(18) SEQ ID NO: 18
YXEGTFTSDYSIX$_2$LDKIAQK(γ-Glu-Palmitoyl)AFVQWLIAGGPSSGAPPPS-$NH_2$
(19) SEQ ID NO: 19
YX$_2$EGTFTSDYSIXLDKIAQK(γ-Glu-Palmitoyl)AFVQWLIAGGPSSGAPPPS-$NH_2$
(20) SEQ ID NO: 20
YX$_1$EGTFTSDYSIX$_2$LDKIAQK(γ-Glu-Palmitoyl)AFVQWLIAGGPSSGAPPPS-$NH_2$
(21) SEQ ID NO: 21
YX$_2$EGTFTSDYSIX$_1$LDKIAQK(γ-Glu-Palmitoyl)AFVQWLIAGGPSSGAPPPS-$NH_2$

Wherein:

X= 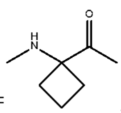 ; X$_1$= 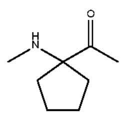

; X$_2$= Aib.

The present invention also discloses a method for preparing the GIP and GLP-1 dual agonist polypeptide compound. The method is characterized by comprising the following steps:

(1) taking resin, activating, and gradually coupling amino acid to obtain a peptide resin; and
(2) taking the peptide resin, and carrying out side chain splicing, splitting and purifying to obtain side chain protected polypeptide.

The present invention also discloses a pharmaceutically acceptable salt of the GIP and GLP-1 dual agonist polypeptide compound. The GIP and GLP-1 dual agonist polypeptide compound is the polypeptide compound as described in the technical solution, and the C-terminal amino acid of the polypeptide compound is amidated as C-terminal primary amide or the pharmaceutically acceptable salt is amidated.

For the pharmaceutically acceptable salt of the GIP and GLP-1 dual agonist polypeptide compound of the present invention, the preferred technical solution is: the salt is a salt formed by GIP and GLP-1 dual agonist polypeptide compound and one of the following compounds: hydrochloric acid, hydrobromic acid, hydroiodic acid, sulfuric acid, pyrosulfuric acid, phosphoric acid, nitric acid, methanesulfonic acid, ethanesulfonic acid, benzenesulfonic acid, p-toluenesulfonic acid, formic acid, acetic acid, acetoacetic acid, pyruvic acid, trifluoroacetic acid, propionic acid, butyric acid, caproic acid, heptanoic acid, undecanoic acid, lauric acid, benzoic acid, salicylic acid, 2-(4-hydroxybenzoyl)benzoic acid, camphoric acid, cinnamic acid, cyclopentanepropionic acid, digluconic acid, 3-hydroxy-2-naphthoic acid, niacin, pamoic acid, pectinic acid, persulfuric acid, 3-phenylpropionic acid, picric acid, pivalic acid, 2-hydroxyethanesulfonic acid, itaconic acid, sulfamic acid, trifluoromethanesulfonic acid, dodecyl sulfuric acid, 2 - naphthalene sulfonic acid, naphthalenedisulfonic acid, camphor sulfonic acid, citric acid, tartaric acid, stearic acid, lactic acid, oxalic acid, malonic acid, succinic acid, malic acid, adipic acid, alginic acid, maleic acid, fumaric acid, D-gluconic acid, mandelic acid, ascorbic acid,

glucoheptanoic acid, glycerophosphoric acid, aspartic acid, sulfosalicylic acid, hemisulfuric acid or thiocyanic acid.

The present invention also discloses a pharmaceutical composition of the GIP and GLP-1 dual agonist polypeptide compound. The pharmaceutical composition is characterized by being prepared by taking the GIP and GLP-1 dual agonist polypeptide compound described in any one of the above technical solutions as an effective raw material or taking a pharmaceutically acceptable salt of any one GIP and GLP-1 dual agonist polypeptide compound in the technical solution as an effective raw material and adding a pharmaceutically acceptable carrier or diluent.

The pharmaceutically acceptable salt of the GIP and GLP-1 dual agonist polypeptide compound is prepared by taking the GIP and GLP-1 dual agonist polypeptide compound and the above compound as raw materials and adopting a conventional method disclosed in the prior art.

The present invention also discloses a medicament prepared from the GIP and GLP-1 dual agonist polypeptide compound described in any one of the above technical solutions. The medicament is in the form of a tablet, a capsule, an elixir, syrup, a lozenge, an inhalant, a spray, an injection, a film, a patch, powder, a granule, a block, an emulsion, a suppository or a compound preparation in pharmacy, and the medicament is prepared from the GIP and GLP-1 dual agonist polypeptide compound and a pharmaceutically acceptable pharmaceutic adjuvant, carrier or diluent. The medicament can be prepared according to a conventional method in the prior art.

According to the application of the GIP and GLP-1 dual agonist polypeptide compound in the present invention, the GIP and GLP-1 dual agonist polypeptide compound can be used as an effective raw material for preparing a medicine for treating or preventing diabetes mellitus or preparing a weight-losing medicine.

According to the pharmaceutical application of the GIP and GLP-1 dual agonist polypeptide compound in the present invention, the GIP and GLP-1 dual agonist polypeptide compound can be used as an effective raw material for preparing a medicine for treating or preventing diabetes mellitus or preparing a weight-losing medicine.

The pharmaceutical composition and the medicament of the GIP and GLP-1 dual agonist polypeptide compound in the present invention can be used as an effective raw material for preparing a medicine for treating and preventing diabetes mellitus or a weight-losing medicine.

In this specification, the meanings of the following abbreviations are shown in the table below:

| Abbreviation | Meaning |
|---|---|
| DCM | Dichloromethane |
| DMF | N-methylpyrrolidone |
| HBTU | Benzotriazole-N,N,N',N'-tetramethylurea hexafluorophosphate |
| HOBt | 1-hydroxy-benzotriazole |
| DIEA/DIPEA | N,N'-diisopropylethylamine |
| Fmoc | N-9-fluorenemethoxycarbonyl |
| EDT | Ethanedithiol |
| HPLC | High performance liquid chromatography |
| TFA | Trifluoroacetate |
| tBu | Tert-butyl |
| DMSO | Dimethyl sulfoxide |
| Alloc | Allyloxycarbonyl |

Compared with the prior art, the present invention has the following beneficial effects:

The present invention discloses a novel GIP and GLP-1 dual agonist polypeptide compound. The polypeptide compound has the effects of reducing blood glucose and losing weight at the same time, and can be used as the effective raw material for preparing the medicine for treating or preventing diabetes or the weight-losing medicine. Based on animal energy consumption data, the polypeptide compound has the effect of losing weight of patients, has balanced co-agonist activity on GIP and GLP-1 receptors and selectivity on glucagon and GLP-2 receptors, has the characteristic of low immunogenicity and the pharmacokinetic (PK) characteristics supporting once-weekly dosing, and is suitable for serving as an active ingredient of a medicine for treating diabetes and obesity.

The GIP and GLP-1 dual agonist polypeptide compound prepared by the method has good activity of reducing blood glucose and slowing weight gain, and is long in effect time, high in yield, short in synthesis period, easy in crude product purification, low in production cost and easy for automatic industrial production.

BRIEF DESCRIPTION OF THE DRAWINGS

FIG. 1 and FIG. 2 are diagrams showing the effect of single administration of each test substance on random blood glucose and $AUC_{0\text{-}24hGlu}$ Glu in *ob*/*ob* mice ($\overline{X}\pm s$, n=7);

FIG. 3 is a diagram showing the effect of single administration of each test substance on AUC0-24h Glu in ob/ob mice ($\overline{X}\pm s$, n=7) (data in the diagram is AUC0-24h Glu decrease rate).

**DETAILED DESCRIPTION**

The present invention is described with the following embodiments, but these embodiments do not constitute any limitation to the rights of the present invention.

**Example 1,** Synthesis of polypeptide compound of SEQ ID NO: 1:

YVEGTFTSDYSIVLDKIAQK(HOOC-$(CH_2)_{16}$-CO-γ-Glu-AEEA-AEEA)AFVQWLIAGGPSSGAPPPS-$NH_2$

1. Synthesis of peptide chain

1.1 Swelling of resin

10 g of Fmoc-Rink Amide AM Resin (with the substitution degree of 0.35 mmol/g) was weighed and swelled for 30 min with 100 mL of DCM, suction filtration was conducted to remove DCM, swelling was conducted for 30 min with 100 mL of DMF, and the resin was washed with 100 mL of DMF and 100 mL of DCM respectively.

1.2 Synthesis of Fmoc-Ser(tBu)- Rink Amide AM Resin

Fmoc- Ser(tBu)-OH (8 mmol), HOBT (16 mmol) and DIC (16 mmol) were dissolved in 100 mL of DMF, then the solution was added into resin obtained in the previous step to react for 2 hours, after reaction ended, the reaction solution was filtered out, and the resin was washed 3 times with 100 mL of DCM and 100 mL of DMF respectively.

1.3 Removal of Fmoc protecting group

A25% piperidine/DMF (V/V) solution containing 0.1 M of HOBt was added into the washed resin to remove Fmoc, and after the reaction ended, the resin was washed 3 times with 100 mL of DCM and 100 mL of DMF respectively.

1.4 Extension of peptide chain

According to the sequence, the steps of deprotection and coupling were repeated to sequentially bind corresponding amino acids, and the corresponding amino acids were sequentially bound until peptide chain synthesis was completed, to obtain the peptide resin.

The $20^{th}$ K could adopt a Fmoc-Lys(Alloc)-OH 或 Fmoc-Lys(Dde)-OH or Fmoc-Lys(iVdde)-OH or Fmoc-Lys(Mtt)-OH or Fmoc-Lys(Boc)-OH protection strategy. In this example, the Fmoc-Lys(Alloc)-OH protection strategy was adopted.

1.5 Splicing of side chains

The obtained peptide resin was put into a reaction bottle, and an Alloc protecting group was removed by using $Pd(PPh_3)_4$ under the condition that $PhSiH_3$ was used as a scavenger; and a 20-site Lys side chain was synthesized by adopting an Fmoc/t-Bu strategy, or Oct(OtBu)-γ-Glu(OtBu)-AEEA-AEEA-Osu fragments were directly coupled to obtain the fully-protected peptide resin.

1.6 Cleavage of peptide resin

Trifluoroacetic acid was measured to a reactor and cooled to -10°C to 0°C, triisopropylsilane, 1, 2-dithioglycol and purified water were added and stirred to be uniformly mixed. Peptide resin was slowly added, heated to 20-30°C, and a cracking reaction was conducted for 115-125 min. After the reaction ended, the resin was filtered out, the filtered resin was washed with M×8×20% ml of TFA, the filtered solution and the washing solution were completely transferred into M×8×1.2×4 ml of diethyl ether, stirred for 5-10 min, kept standing to precipitate for 15 min or more. The precipitated turbid liquid was added into a centrifugal machine, and solids were centrifuged and collected; the solids were washed with diethyl ether six times, and the amount of diethyl ether used each time was not less than 5 L. The solids were subjected to vacuum drying for 6-10 h at the temperature of 20-35°C, and crude peptide was obtained.

1.7 Purification of crude peptide

Purification was conducted through preparative liquid chromatography, and the chromatographic conditions were that a C18 column (100 mm×250 mm, 10 μm) was adopted; a mobile phase A was 0.1% TFA/water (V/V), and a mobile phase B was 0.1% TFA/acetonitrile (V/V); the mobile phase gradient was 20%-60% of the mobile phase B, and the time was 60 min; the flow velocity was 200 mL/min, the detection wavelength was 214 nm, fractions with the purity larger than 98.0% were collected, and 1.36 g of samples were obtained through freeze drying after rotary evaporation and concentration.

**Example 2,** Synthesis of polypeptide compound of SEQ ID NO: 2:

YXEGTFTSDYSIXLDKIAQK(HOOC-$(CH_2)_{16}$-CO-γ-Glu-AEEA-AEEA)AFVQWLIAGGPSSGAPPPS-$NH_2$

X= 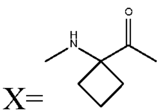

The synthesis procedures were the same as those in Example 1, and the collected solution was freeze-dried to obtain a purified product in a mass of 1.45 g.

**Example 3,** Synthesis of polypeptide compound of SEQ ID NO: 3:

YXEGTFTSDYSIXLDKIAQK(HOOC-$(CH_2)_{16}$-CO-γ-Glu-AEEA-AEEA)AFVQWLIAGGPSSGAPPPS-$NH_2$

X= 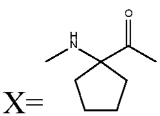

The synthesis procedures were the same as those in Example 1, and the collected solution was freeze-dried to obtain a purified product in a mass of 1.51 g.

**Example 4,** Synthesis of polypeptide compound of SEQ ID NO: 4:

YAEGTFTSDYSIALDKIAQK(HOOC-$(CH_2)_{16}$-CO-γ-Glu-AEEA-AEEA)AFVQWLIAGGPSSGAPPPS-$NH_2$

The synthesis procedures were the same as those in Example 1, and the collected solution was freeze-dried

to obtain a purified product in a mass of 1.18 g.

**Example 5,** Synthesis of polypeptide compound of SEQ ID NO: 5:

YAEGTFTSDYSIAibLDKIAQK(HOOC-$(CH_2)_{16}$-CO-$\gamma$-Glu-AEEA-AEEA)AFVQWLIAGGPSSGAPPPS-$NH_2$

The synthesis procedures were the same as those in Example 1, and the collected solution was freeze-dried to obtain a purified product in a mass of 1.22 g.

**Example 6,** Synthesis of polypeptide compound of SEQ ID NO: 6:

YAibEGTFTSDYSIALDKIAQK(HOOC-$(CH_2)_{16}$-CO-$\gamma$-Glu-AEEA-AEEA)AFVQWLIAGGPSSGAPPPS-$NH_2$

The synthesis procedures were the same as those in Example 1, and the collected solution was freeze-dried to obtain a purified product in a mass of 1.97 g.

**Example 7,** Synthesis of polypeptide compound of SEQ ID NO: 7:

YX1EGTFTSDYSIX2LDKIAQK(HOOC-$(CH_2)_{16}$-CO-$\gamma$-Glu-AEEA-AEEA)AFVQWLIAGGPSSGAPPPS-$NH_2$

$X_1$= 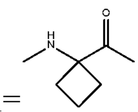

$X_2$= Aib

The synthesis procedures were the same as those in Example 1, and the collected solution was freeze-dried to obtain a purified product in a mass of 1.55 g.

**Example 8,** Synthesis of polypeptide compound of SEQ ID NO: 8:

Y$X_1$EGTFTSDYSI$X_2$LDKIAQK(HOOC-$(CH_2)_{16}$-CO-$\gamma$-Glu-AEEA-AEEA)AFVQWLIAGGPSSGAPPPS-$NH_2$

$X_1$= Aib

$X_2$= 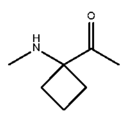

The synthesis procedures were the same as those in Example 1, and the collected solution was freeze-dried to obtain a purified product in a mass of 1.01 g.

**Example 9,** Synthesis of polypeptide compound of SEQ ID NO: 9:

Y$X_1$EGTFTSDYSI$X_2$LDKIAQK(HOOC-$(CH_2)_{16}$-CO-γ-Glu-AEEA-AEEA)AFVQWLIAGGPSSGAPPPS-$NH_2$

$X_1$= 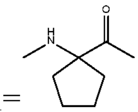

$X_2$= Aib

The synthesis procedures were the same as those in Example 1, and the collected solution was freeze-dried to obtain a purified product in a mass of 1.64 g.

**Example 10,** Synthesis of polypeptide compound of SEQ ID NO: 10:

Y$X_1$EGTFTSDYSI$X_2$LDKIAQK(HOOC-$(CH_2)_{16}$-CO-γ-Glu-AEEA-AEEA)AFVQWLIAGGPSSGAPPPS-$NH_2$

$X_1$= Aib

$X_2$= 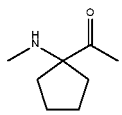

The synthesis procedures were the same as those in Example 1, and the collected solution was freeze-dried to obtain a purified product in a mass of 1.81 g.

**Example 11,** Synthesis of polypeptide compound of SEQ ID NO: 11: YAibEGTFTSDYSIAibLDKIAQK(γ-Glu-Palmitoyl)AFVQWLIAGGPSSGAPPPS-$NH_2$ The synthesis procedures were the same as those in Example 1, and the collected solution was freeze-dried to obtain a purified product in a mass of 1.84 g.

**Example 12,** Synthesis of polypeptide compound of SEQ ID NO: 12: YVEGTFTSDYSIVLDKIAQK(γ-Glu-Palmitoyl)AFVQWLIAGGPSSGAPPPS-$NH_2$

The synthesis procedures were the same as those in Example 1, and the collected solution was freeze-dried to obtain a purified product in a mass of 1.12 g.

**Example 13,** Synthesis of polypeptide compound of SEQ ID NO: 13: YXEGTFTSDYSIXLDKIAQK(γ-Glu-Palmitoyl)AFVQWLIAGGPSSGAPPPS-$NH_2$

X= 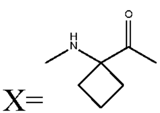

The synthesis procedures were the same as those in Example 1, and the collected solution was freeze-dried to obtain a purified product in a mass of 1.07 g.

**Example 14,** Synthesis of polypeptide compound of SEQ ID NO: 14: YXEGTFTSDYSIXLDKIAQK(γ-Glu-Palmitoyl)AFVQWLIAGGPSSGAPPPS-$NH_2$

X= 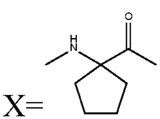

The synthesis procedures were the same as those in Example 1, and the collected solution was freeze-dried to obtain a purified product in a mass of 1.89 g.

**Example 15,** Synthesis of polypeptide compound of SEQ ID NO: 15: YAEGTFTSDYSIALDKIAQK(γ-Glu-Palmitoyl)AFVQWLIAGGPSSGAPPPS-$NH_2$

The synthesis procedures were the same as those in Example 1, and the collected solution was freeze-dried to obtain a purified product in a mass of 1.81 g.

**Example 16,** Synthesis of polypeptide compound of SEQ ID NO: 16: YAEGTFTSDYSIAibLDKIAQK(γ-Glu-Palmitoyl)AFVQWLIAGGPSSGAPPPS-$NH_2$

The synthesis procedures were the same as those in Example 1, and the collected solution was freeze-dried to obtain a purified product in a mass of 1.71 g.

**Example 17,** Synthesis of polypeptide compound of SEQ ID NO: 17: YAibEGTFTSDYSIALDKIAQK(γ-Glu-Palmitoyl)AFVQWLIAGGPSSGAPPPS-$NH_2$

The synthesis procedures were the same as those in Example 1, and the collected solution was freeze-dried to obtain a purified product in a mass of 1.53 g.

**Example 18,** Synthesis of polypeptide compound of SEQ ID NO: 18: YX1EGTFTSDYSIX2LDKIAQK(γ-Glu-Palmitoyl)AFVQWLIAGGPSSGAPPPS-$NH_2$

$X_1$= 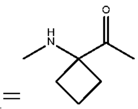

$X_2$= Aib

The synthesis procedures were the same as those in Example 1, and the collected solution was freeze-dried to obtain a purified product in a mass of 1.25 g.

**Example 19,** Synthesis of polypeptide compound of SEQ ID NO: 19: $YX_1EGTFTSDYSIX_2LDKIAQK$(γ-Glu-Palmitoyl)AFVQWLIAGGPSSGAPPPS-$NH_2$

$X_1$= Aib

$X_2$= 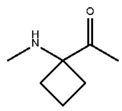

The synthesis procedures were the same as those in Example 1, and the collected solution was freeze-dried to obtain a purified product in a mass of 0.93 g.

**Example 20,** Synthesis of polypeptide compound of SEQ ID NO: 20: $YX_1EGTFTSDYSIX_2LDKIAQK$(γ-Glu-Palmitoyl)AFVQWLIAGGPSSGAPPPS-$NH_2$

$X_1$= 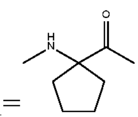

$X_2$= Aib

The synthesis procedures were the same as those in Example 1, and the collected solution was freeze-dried to obtain a purified product in a mass of 0.96g.

**Example 21,** Synthesis of polypeptide compound of SEQ ID NO: 15: $YX_1EGTFTSDYSIX_2LDKIAQK$(γ-Glu-Palmitoyl)AFVQWLIAGGPSSGAPPPS-$NH_2$

$X_1$= Aib

$X_2$= 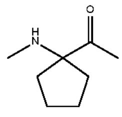

The synthesis procedures were the same as those in Example 1, and the collected solution was freeze-dried to obtain a purified product in a mass of 1.47 g.

Example 22: the relevant pharmacological experimental methods and results of a GIP and GLP-1 dual agonist polypeptide compound (hereinafter referred to as a polypeptide compound):

1. **GLP-1 and GIP receptor agonist activity of** polypeptide compound

HEK293 cells were co-transfected with cDNA encoding GLP-1R or GIPR. In a test for determining the compound, the cells were inoculated in a 96-well plate 2 h in advance, the polypeptide compound was dissolved with DMSO, and diluted to different multiples with a culture medium containing 0.1% bovine serum albumin and added to the co-transfected cells. After the cells were incubated for 20 min, fluorescence values were determined through microplate reader of an ELISA kit of the Cisbo Company, a standard curve was established to convert the fluorescence values into corresponding cAMP values, and the $EC_{50}$ value of the compound was calculated through nonlinear regression of Graphpad Prism 5.0 software.

Table 1 Agonist activity of polypeptide compound on GLP-1R and GIPR

| **Peptides** | **mGLP-1R** | **mGIPR** | **Peptides** | **mGLP-1R** | **mGIPR** |
|---|---|---|---|---|---|
| | **$EC_{50}$ (nM)** | **EC50 (nM)** | | **EC50 (nM)** | **EC50 (nM)** |
| Semaglutide | 0.0076 | 68 | GIP | \ | 0.0011 |
| GLP-1 | 0.00127 | \ | SEQ.ID NO:11 | 0.02427 | 0.00632 |
| SEQ.ID NO:1 | 0.01457 | 0.00068 | SEQ.ID NO:12 | 0.05613 | 0.00879 |
| SEQ.ID NO:2 | 0.00613 | 0.00086 | SEQ.ID NO:13 | 0.05396 | 0.02250 |
| SEQ.ID NO:3 | 0.00396 | 0.00149 | SEQ.ID NO:14 | 0.03109 | 0.05165 |
| SEQ.ID NO:4 | 0.00149 | 0.00521 | SEQ.ID NO:15 | 0.04290 | 0.05320 |
| SEQ.ID NO:5 | 0.00299 | 0.00640 | SEQ.ID NO:16 | 0.04250 | 0.00965 |
| SEQ.ID NO:6 | 0.00258 | 0.00929 | SEQ.ID NO:17 | 0.04541 | 0.00972 |
| SEQ.ID NO:7 | 0.00549 | 0.00766 | SEQ.ID NO:18 | 0.00902 | 0.00833 |
| SEQ.ID NO:8 | 0.00805 | 0.00858 | SEQ.ID NO:19 | 0.01189 | 0.00791 |
| SEQ.ID NO:9 | 0.00941 | 0.00698 | SEQ.ID NO:20 | 0.01587 | 0.00591 |
| SEQ.ID NO:10 | 0.00889 | 0.00811 | SEQ.ID NO:21 | 0.01783 | 0.00807 |

As shown in Table 1, the agonist activity of all polypeptide compounds to GLP-1R still kept a high degree of agonist activity, and meanwhile kept a quite high degree of agonist activity to GIPR, and modified non-natural amino acids and side chain groups did not produce great influence on the agonist activity.

2. Blood glucose reducing experiment of polypeptide compound

Male ob/ob mice were fed with high-fat feed in a single cage after weaning, blood glucose was predicted after 6-7 weeks, and the mice were divided into a model control group, a polypeptide compound group (selecting 9 compounds), a positive control Liraglutide group and a positive control Semaglutide group according to random weight, random blood glucose and fasting blood glucose. The mice in each test group and the positive control group received a single subcutaneous injection of test substances or positive drugs Liraglutide and Semaglutide at different doses, the mice in the model control group received a single subcutaneous injection of a PBS buffer solution. The mice in each group received random blood glucose value measurement before (0 h) administration and 2, 4, 6, 10 and 24 h after administration, and then the time for measuring the random blood glucose was adjusted and prolonged to 34, 48, 58 or 72 h after administration according to the blood glucose condition of the mice in each group. Meanwhile, the random weight of the mice in each group before (0 h) administration and 24 h after administration was weighed, and then the random weight of the mice corresponding to the random blood glucose measurement condition 48 or 72 h after administration was weighed. The blood glucose decrease rate, the blood glucose curve area ($AUC_{0\text{-}24h\ Glu}$) within 24 h after administration and the decrease rate were calculated according to the following formula.

$$\text{Blood glucose decrease rate} = (\text{blood glucose in the model control group} - \text{blood glucose in the administration group})/\text{blood glucose in the model control group} \times 100\%$$

$$AUC_{0\text{-}24h\ Glu}(mmol/Lh{\cdot}r) = (BG0+BG2) + (BG2+BG4) + (BG4+BG6) + (BG6+BG10)\times 2 + (BG10+BG24)\times 7$$

BG0, BG2, BG4, BG6, BG10 and BG24 represented the blood glucose values before administration (0h) and 2, 4, 6, 10 and 24 h after administration respectively.

$$AUC_{0\text{-}24h\ Glu}(mmol/Lh{\cdot}r)\ \text{decrease rate} = (\text{model control group } AUC_{0\text{-}24h\ Glu} - \text{administration group } AUC_{0\text{-}24h\ Glu}/\text{model control group } AUC_{0\text{-}24h\ Glu}\times 100\%$$

Experiments were carried out twice, at an interval of 8-10 days. There were 23 groups in each experiment, and the experiment grouping and dosage setting conditions were shown in Table 2.

Table 2 Grouping of experimental animals before administration ($\overline{X}\pm s$)

| Groups | Dosage (μg/kg) | Random body weight (g) | Random blood glucose (mmol/L) | Fasting blood glucose (mmol/L) |
|---|---|---|---|---|
| Model control | - | 42.0±0.8 | 20.23±1.01 | 18.94±0.99 |
| GIP | 100 | 42.5±0.8 | 20.36±0.74 | 18.97±0.64 |
| GLP-1 | 100 | 42.3±0.7 | 20.30±1.05 | 19.34±1.07 |
| Liraglutide | 100 | 42.1±1.1 | 20.14±0.98 | 18.86±1.28 |
| Semaglutide | 100 | 42.3±1.0 | 20.71±1.14 | 18.83±0.85 |
| SEQ.ID NO:1 | 100 | 42.2±0.5 | 20.31±0.99 | 18.59±0.68 |
| SEQ.ID NO:2 | 100 | 42.3±0.8 | 20.62±1.03 | 18.76±0.75 |
| SEQ.ID NO:3 | 100 | 42.1±0.5 | 20.45±0.91 | 18.72±1.02 |
| SEQ.ID NO:4 | 100 | 42.6±1.0 | 20.43±0.68 | 18.82±0.75 |
| SEQ.ID NO:5 | 100 | 42.4±0.9 | 20.57±0.84 | 18.57±1.14 |
| SEQ.ID NO:6 | 100 | 42.6±0.6 | 20.64±0.77 | 19.15±0.80 |
| SEQ.ID NO:7 | 100 | 42.7±1.0 | 20.64±0.99 | 18.97±0.69 |
| SEQ.ID NO:8 | 100 | 42.8±0.7 | 20.36±0.87 | 18.84±0.78 |
| SEQ.ID NO:9 | 100 | 42.4±0.8 | 20.24±0.95 | 19.02±0.73 |

As shown in FIG. 1 to FIG. 3, blood glucose reducing experiment results showed that, when the administration concentration of the polypeptide compound was 100 μg/kg, most of the blood glucose reducing effects were equivalent to those of Semaglutide and GIP., and especially the blood glucose reducing effect of SEQ. ID NO: 2 was almost consistent with that of Semaglutide.

3. Effect of polypeptide compound on random weight of ob/ob mice

As shown in Tables 3 to 5, single subcutaneous injection administration of 100 g/kg of GIP and 100 g/kg of GLP-1 and 300 g/kg of GLP-1 had no obvious influence on the random weight and the variable quantity of the ob/ob mice. After a single subcutaneous injection administration of 100 g/kg of Liraglutide and Semaglutide groups, the random weight and the variable quantity of the ob/ob mice could be significantly reduced in 24 h and 48 h following the administration. After a single subcutaneous injection administration of 300 g/kg of SEQ. ID NO: 1-9, the random weight and the variable quantity of the ob/ob mice could be significantly reduced in 24 h, 48 h and 72 h following the administration. After a single subcutaneous injection administration of 100 g/kg of SEQ. ID NO: 2, the random weight variable quantity of the ob/ob mice could be significantly reduced in 24 hours following the administration. After a single subcutaneous injection administration of 300 g/kg of SEQ. ID NO: 9, the random weight variable quantity of the ob/ob mice could be

significantly reduced in 24 h and 48 h following the administration; and the effect of SEQ. ID NO:2 was basically equivalent to that of the equal dose of Semaglutide. Therefore, the single subcutaneous injection administration of the GIP and GLP-1 dual agonist compound could significantly reduce the random blood glucose of the type 2 diabetes ob/ob mice, wherein the effect of SEQ. ID NO: 2 was comparable to that of the equal dose of Semaglutide. After long-term administration, all polypeptide compounds showed a better weight control effect.

Table 3: Effect of single administration of each test substance on random blood glucose and $AUC_{0\text{-}24h\ Glu}$ in *ob/ob* mice ($\overline{X}\pm s$, n=7)

| Groups | Dosage (μg/kg) | Blood glucose at different time after administration (mmol/L) | | | | | | | | $AUC_{0\text{-}24h\ Glu}$ (mmol/L hr) |
|---|---|---|---|---|---|---|---|---|---|---|
| | | 0h | 2h | 4h | 6h | 10h | 24h | 34h | 48h | |
| Model control | - | 20.23±1.01 | 19.72±1.86 | 21.43±1.92 | 21.12±1.07 | 22.06±1.83 | 20.89±1.08 | 21.19±1.14 | 19.71±2.31 | 510.66±24.79 |
| GIP | 100 | 20.36±0.74 | 12.63±1.70 | 12.46±1.66 | 13.71±0.86 | 14.16±1.24 | 17.84±0.87 | - | - | 363.99±14.21 |
| GLP-1 | 100 | 20.30±1.05 | 12.06±1.36 | 11.79±0.84 | 13.02±0.86 | 14.03±2.41 | 18.04±0.71 | - | - | 359.61±18.59 |
| Liraglutide | 100 | 20.14±0.98 | 11.56±1.12 | 10.34±1.30 | 10.63±0.84 | 12.03±0.81 | 17.71±1.08 | - | - | 328.07±19.12 |
| Semaglutide | 100 | 20.71±1.14 | 9.12±0.84 | 8.96±1.40 | 8.32±0.88 | 8.29±0.86 | 8.49±1.12 | 9.12±0.83 | 17.83±1.42 | 215.87±18.67 |
| SEQ.ID NO:1 | 100 | 20.31±0.99 | 10.12±0.87 | 9.94±0.86 | 9.83±0.87 | 10.76±1.12 | 11.89±2.12 | 13.93±1.01 | 17.32±0.89 | 269.99±19.45 |
| SEQ.ID NO:2 | 100 | 20.62±1.03 | 9.14±0.83 | 8.95±0.84 | 8.33±1.42 | 8.31±1.02 | 8.51±0.86 | 8.95±0.87 | 18.01±1.12 | 216.15±18.86 |
| SEQ.ID NO:3 | 100 | 20.45±0.91 | 10.11±1.01 | 9.52±1.02 | 9.63±2.42 | 9.76±1.13 | 10.01±1.42 | 11.56±0.97 | 17.06±1.21 | 246.51±24.91 |
| SEQ.ID NO:4 | 100 | 20.43±0.68 | 10.21±1.06 | 9.89±0.83 | 9.63±0.95 | 9.86±0.96 | 11.01±0.83 | 14.56±1.42 | 17.32±0.83 | 255.33±20.13 |
| SEQ.ID NO:5 | 100 | 20.57±0.84 | 10.12±1.20 | 9.54±0.84 | 9.43±0.86 | 9.76±0.96 | 10.89±1.43 | 13.33±2.10 | 17.32±1.12 | 252.25±19.68 |
| SEQ.ID NO:6 | 100 | 20.64±0.77 | 9.76±0.83 | 8.96±0.77 | 8.55±0.96 | 9.93±0.76 | 10.53±1.21 | 13.13±2.21 | 17.79±0.86 | 246.81±18.95 |
| SEQ.ID NO:7 | 100 | 20.64±0.99 | 10.11±0.88 | 9.58±0.97 | 9.66±0.84 | 9.76±0.79 | 10.93±0.86 | 13.54±1.24 | 17.34±1.20 | 253.35±18.65 |
| SEQ.ID NO:8 | 100 | 20.36±0.87 | 10.56±0.99 | 9.99±0.86 | 9.83±0.85 | 9.54±1.12 | 11.95±1.26 | 14.33±1.43 | 17.16±1.52 | 260.46±21.31 |
| SEQ.ID NO: 9 | 100 | 20.24±0.95 | 9.85±0.89 | 9.51±0.97 | 8.92±0.86 | 8.93±0.86 | 10.57±0.83 | 12.26±0.94 | 17.28±1.28 | 240.08±18.97 |

Table 4: Decrease rate of random blood glucose and $AUC_{0\text{-}24h\ Glu}$ in *ob*/*ob* mice after single administration of each test substance ($\bar{X}\pm s$, n=7)

| Groups | Dosage (μg/k g) | Decrease rate of blood glucose at different time after administration (%) | | | | | | | $AUC_{0\text{-}24h\ Glu}$ Decrease rate (%) |
|---|---|---|---|---|---|---|---|---|---|
| | | 2h | 4h | 6h | 10h | 24h | 34h | 48h | |
| GIP | 100 | 38.0 | 38.8 | 32.7 | 30.5 | 12.4 | - | - | 28.7 |
| GLP-1 | 100 | 40.6 | 41.9 | 35.9 | 30.9 | 11.1 | - | - | 29.6 |
| Liraglutide | 100 | 42.6 | 48.7 | 47.2 | 40.3 | 12.1 | - | - | 35.8 |
| Semaglutide | 100 | 56.0 | 56.7 | 59.8 | 60.0 | 59.0 | 56.0 | 13.9 | 57.7 |
| SEQ.ID NO:1 | 100 | 50.2 | 51.1 | 51.6 | 47.0 | 41.5 | 31.4 | 14.7 | 47.1 |
| SEQ.ID NO:2 | 100 | 55.7 | 56.6 | 59.6 | 59.7 | 58.7 | 56.6 | 12.7 | 57.7 |
| SEQ.ID NO:3 | 100 | 50.6 | 53.4 | 52.9 | 52.3 | 51.1 | 43.5 | 16.6 | 51.7 |
| SEQ.ID NO:4 | 100 | 50.0 | 51.6 | 52.9 | 51.7 | 46.1 | 28.7 | 15.2 | 50.0 |
| SEQ.ID NO:5 | 100 | 50.8 | 53.6 | 54.2 | 52.6 | 47.1 | 35.2 | 15.8 | 50.6 |
| SEQ.ID NO:6 | 100 | 52.7 | 56.6 | 58.6 | 51.9 | 49.0 | 36.4 | 13.8 | 51.7 |
| SEQ.ID NO:7 | 100 | 51.0 | 53.6 | 53.2 | 52.7 | 47.0 | 34.4 | 16.0 | 50.4 |
| SEQ.ID NO:8 | 100 | 48.1 | 50.9 | 51.7 | 53.1 | 41.3 | 29.6 | 15.7 | 49.0 |
| SEQ.ID NO:9 | 100 | 51.3 | 53.0 | 55.9 | 55.9 | 47.8 | 39.4 | 14.6 | 53.0 |

Table 5: Effect of single administration of each test substance on random body weight and variable quantity in *ob*/*ob* mice ($X\pm s$,n=7)

| Groups | Dosage (μg/kg) | Random body weight at different time after administration (g) | | | Changes of random body weight at different time after administration (g) | | |
|---|---|---|---|---|---|---|---|
| | | 0h | 24h | 48h | 0h | 24h | 48h |
| Model control | - | 42.9±0.8 | 43.5±0.8 | 43.6±0.7 | 0.0±0.0 | 0.6±0.1 | 0.7±0.1 |
| GIP | 100 | 43.5±0.8 | 43.6±0.8 | - | 0.0±0.0 | 0.1±0.1 | - |
| GLP-1 | 100 | 43.2±0.7 | 43.1±0.7 | - | 0.0±0.0 | -0.1±0.1 | - |
| Liraglutide | 100 | 43.0±1.1 | 42.6±1.1 | - | 0.0±0.0 | -0.5±0.1 | - |
| Semaglutide | 100 | 43.7±1.0 | 40.7±1.0 | 41.7±1.0 | 0.0±0.0 | -2.9±0.2 | -1.9±0.2 |
| SEQ.ID NO:1 | 100 | 43.2±0.5 | 42.0±0.5 | 42.7±0.6 | 0.0±0.0 | -1.1±0.2 | -0.6±0.1 |
| SEQ.ID NO:2 | 100 | 43.3±0.8 | 40.3±0.8 | 41.3±0.8 | 0.0±0.0 | -2.9±0.2 | -1.9±0.2 |
| SEQ.ID NO:3 | 100 | 43.2±0.5 | 41.6±0.6 | 42.1±0.6 | 0.0±0.0 | -1.5±0.2 | -0.9±0.2 |
| SEQ.ID NO:4 | 100 | 43.6±1.0 | 42.2±1.0 | 42.9±1.1 | 0.0±0.0 | -1.4±0.2 | -0.6±0.1 |
| SEQ.ID NO:5 | 100 | 43.5±0.9 | 42.0±0.9 | 42.9±0.9 | 0.0±0.0 | -1.4±0.2 | -0.6±0.2 |
| SEQ.ID NO:6 | 100 | 43.5±0.6 | 42.1±0.7 | 42.7±0.7 | 0.0±0.0 | -1.5±0.2 | -0.8±0.1 |
| SEQ.ID NO:7 | 100 | 43.7±1.0 | 42.1±1.0 | 43.0±0.9 | 0.0±0.0 | -1.5±0.2 | -0.7±0.1 |
| SEQ.ID NO:8 | 100 | 43.8±0.7 | 42.7±0.7 | 43.2±0.7 | 0.0±0.0 | -1.1±0.2 | -0.6±0.1 |
| SEQ.ID NO:9 | 100 | 43.5±0.8 | 41.5±0.8 | 42.4±0.9 | 0.0±0.0 | -1.9±0.2 | -1.1±0.1 |

## Claims

1. A GIP and GLP-1 dual agonist polypeptide compound, having an amino acid sequence as follows: $YXaa^1EGTFTSDYSIXaa^2LDKIAQXaa^3AFVQWLIAGGPSSGAPPPS$, and the C-terminal amino acid of the polypeptide compound is amidated as C-terminal primary amide; wherein:

$Xaa^1$ is: V, Aib , A,

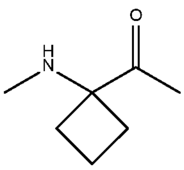 , 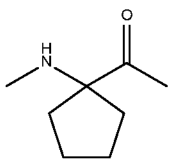 , 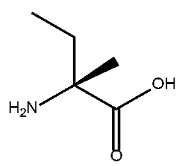 or 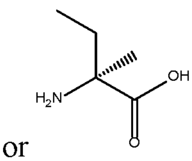 ;

$Xaa^2$ is: V, Aib, A,

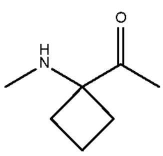 , 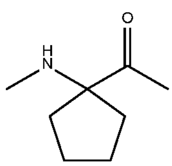 , 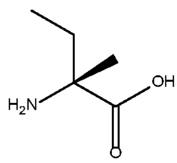 or 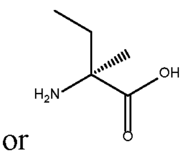 ;

$Xaa^1$ and $Xaa^2$ are the same or different;
$Xaa^3$ is:

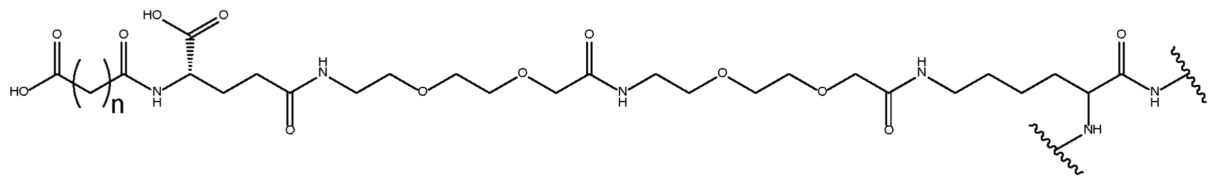

or:

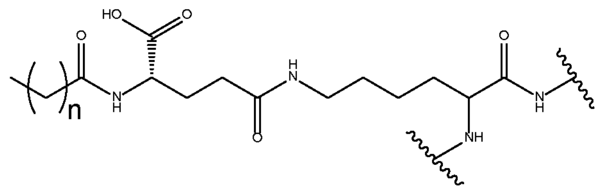

n is a natural number ranging from 5 to 25.

**2.** The compound of claim 1, wherein n is a natural number 16 or 18.

**3.** The compound of claim 1 or 2, having one of the following amino acid sequences:

(1) SEQ ID NO: 1

YVEGTFTSDYSIVLDKIAQK(HOOC-$(CH_2)_{16}$-CO-γ-Glu- AEEA-AEEA)AFVQWLIAGGPSSGAPPPS-$NH_2$

(2) SEQ ID NO: 2

YXEGTFTSDYSIXLDKIAQK(HOOC-$(CH_2)_{16}$-CO-γ-Glu-AEEA-AEEA)AFVQWLIAGGPSSGAPPPS-$NH_2$

(3) SEQ ID NO: 3

$YX_1$EGTFTSDYSI$X_1$LDKIAQK(HOOC-$(CH_2)_{16}$-CO-γ-Glu-AEEA-AEEA)AFVQWLIAGGPSSGAPPPS-$NH_2$

(4) SEQ ID NO: 4

YAEGTFTSDYSIALDKIAQK(HOOC-$(CH_2)_{16}$-CO-γ-Glu-AEEA-AEEA)AFVQWLIAGGPSSGAPPPS-$NH_2$

(5) SEQ ID NO: 5

YAEGTFTSDYSIAibLDKIAQK(HOOC-$(CH_2)_{16}$-CO-γ-Glu-AEEA-AEEA)AFVQWLIAGGPSSGAPPPS-$NH_2$

(6) SEQ ID NO: 6

YAibEGTFTSDYSIALDKIAQK(HOOC-$(CH_2)_{16}$-CO-γ-Glu-AEEA-AEEA)AFVQWLIAGGPSSGAPPPS-$NH_2$

(7) SEQ ID NO: 7

YXEGTFTSDYSI$X_2$LDKIAQK(HOOC-$(CH_2)_{16}$-CO-γ-Glu-AEEA-AEEA)AFVQWLIAGGPSSGAPPPS-$NH_2$

(8) SEQ ID NO: 8

YXEGTFTSDYSI$X_2$LDKIAQK(HOOC-$(CH_2)_{16}$-CO-γ-Glu-AEEA-AEEA)AFVQWLIAGGPSSGAPPPS-$NH_2$

(9) SEQ ID NO: 9

Y$X_1$EGTFTSDYSI$X_2$LDKIAQK(HOOC-$(CH_2)_{16}$-CO-γ-Glu-AEEA-AEEA)AFVQWLIAGGPSSGAPPPS-$NH_2$

(10) SEQ ID NO: 10

Y$X_2$EGTFTSDYSI$X_1$LDKIAQK(HOOC-$(CH_2)_{16}$-CO-γ-Glu-AEEA-AEEA)AFVQWLIAGGPSSGAPPPS-$NH_2$

(11) SEQ ID NO: 11
YAibEGTFTSDYSIAibLDKIAQK(γ-Glu-Palmitoyl)AFVQWLIAGGPSSGAPPPS-$NH_2$
(12) SEQ ID NO: 12
YVEGTFTSDYSIVLDKIAQK(γ-Glu-Palmitoyl)AFVQWLIAGGPSSGAPPPS-$NH_2$
(13) SEQ ID NO: 13
YXEGTFTSDYSIXLDKIAQK(γ-Glu-Palmitoyl)AFVQWLIAGGPSSGAPPPS-$NH_2$
(14) SEQ ID NO: 14
Y$X_1$EGTFTSDYSI$X_1$LDKIAQK(γ-Glu-Palmitoyl)AFVQWLIAGGPSSGAPPPS-$NH_2$
(15) SEQ ID NO: 15
YAEGTFTSDYSIALDKIAQK(γ-Glu-Palmitoyl)AFVQWLIAGGPSSGAPPPS-$NH_2$
(16) SEQ ID NO: 16
YAEGTFTSDYSIAibLDKIAQK(γ-Glu-Palmitoyl)AFVQWLIAGGPSSGAPPPS-$NH_2$
(17) SEQ ID NO: 17
YAibEGTFTSDYSIALDKIAQK(γ-Glu-Palmitoyl)AFVQWLIAGGPSSGAPPPS-$NH_2$
(18) SEQ ID NO: 18
YXEGTFTSDYSI$X_2$LDKIAQK(γ-Glu-Palmitoyl)AFVQWLIAGGPSSGAPPPS-$NH_2$
(19) SEQ ID NO: 19
Y$X_2$EGTFTSDYSIXLDKIAQK(γ-Glu-Palmitoyl)AFVQWLIAGGPSSGAPPPS-$NH_2$
(20) SEQ ID NO: 20
Y$X_1$EGTFTSDYSI$X_2$LDKIAQK(γ-Glu-Palmitoyl)AFVQWLIAGGPSSGAPPPS-$NH_2$
(21) SEQ ID NO: 21
Y$X_2$EGTFTSDYSI$X_1$LDKIAQK(γ-Glu-Palmitoyl)AFVQWLIAGGPSSGAPPPS-$NH_2$

wherein:

X= 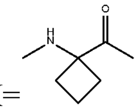 ;

$X_1$= 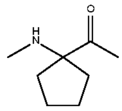

; $X_2$= Aib.

**4.** A method of preparing the GIP and GLP-1 dual agonist polypeptide compound of claim 1, 2 or 3, the method comprising:

(1) taking resin, activating, and gradually coupling amino acid to obtain a peptide resin; and
(2) taking the peptide resin, and carrying out side chain splicing, splitting and purifying to obtain side chain protected polypeptide.

**5.** A pharmaceutically acceptable salt of the GIP and GLP-1 dual agonist polypeptide compound of claim 1 or 2 or 3, wherein a C-terminal amino acid of the polypeptide compound is amidated as C-terminal primary amide or the pharmaceutically acceptable salt is amidated.

**6.** The salt of claim 5, wherein the salt is a salt formed by GIP and GLP-1 dual agonist polypeptide compound and one of the following compounds: hydrochloric acid, hydrobromic acid, hydroiodic acid, sulfuric acid, pyrosulfuric acid, phosphoric acid, nitric acid, methanesulfonic acid, ethanesulfonic acid, benzenesulfonic acid, p-toluenesulfonic acid, formic acid, acetic acid, acetoacetic acid, pyruvic acid, trifluoroacetic acid, propionic acid, butyric acid, caproic acid, heptanoic acid, undecanoic acid, lauric acid, benzoic acid, salicylic acid, 2-(4-hydroxybenzoyl)benzoic acid, camphoric acid, cinnamic acid, cyclopentanepropionic acid, digluconic acid, 3-hydroxy-2-naphthoic acid, niacin, pamoic acid, pectinic acid, persulfuric acid, 3-phenylpropionic acid, picric acid, pivalic acid, 2-hydroxyethanesulfonic acid, itaconic acid, sulfamic acid, trifluoromethanesulfonic acid, dodecyl sulfuric acid, 2 - naphthalene sulfonic acid, naphthalenedisulfonic acid, camphor sulfonic acid, citric acid, tartaric acid, stearic acid, lactic acid, oxalic acid, malonic acid, succinic acid, malic acid, adipic acid, alginic acid, maleic acid, fumaric acid, D-gluconic acid, mandelic acid, ascorbic acid, glucoheptanoic acid, glycerophosphoric acid, aspartic acid, sulfosalicylic acid, hemisulfuric acid or thiocyanic acid.

**7.** A medicament prepared from the GIP and GLP-1 dual agonist polypeptide compound of any one of claims 1-3, the medicament being in the form of a tablet, a capsule, an elixir, syrup, a lozenge, an inhalant, a spray, an injection, a film, a patch, powder, a granule, a block, an emulsion, a suppository or a compound preparation.

**8.** A pharmaceutical composition, comprising the GIP and GLP-1 dual agonist polypeptide compound of any one of claims 1-3 as an effective raw material or a pharmaceutically acceptable salt of claim 4 or 5 as an effective raw material, and a pharmaceutically acceptable carrier or diluent.

**9.** Use of the GIP and GLP-1 dual agonist polypeptide compound of any one of claims 1-3 or the GIP and GLP-1 dual agonist polypeptide compound prepared in the method of claim 4, as an effective raw material, for preparing a medicine for treating or preventing diabetes mellitus or preparing a weight-losing medicine.

**10.** Use of the pharmaceutically acceptable salt of claim 5 or 6, or the medicament of claim 7, or the pharmaceutical composition of claim 8, as an effective raw material, for preparing a medicine for treating or preventing diabetes mellitus or preparing a weight-losing medicine.

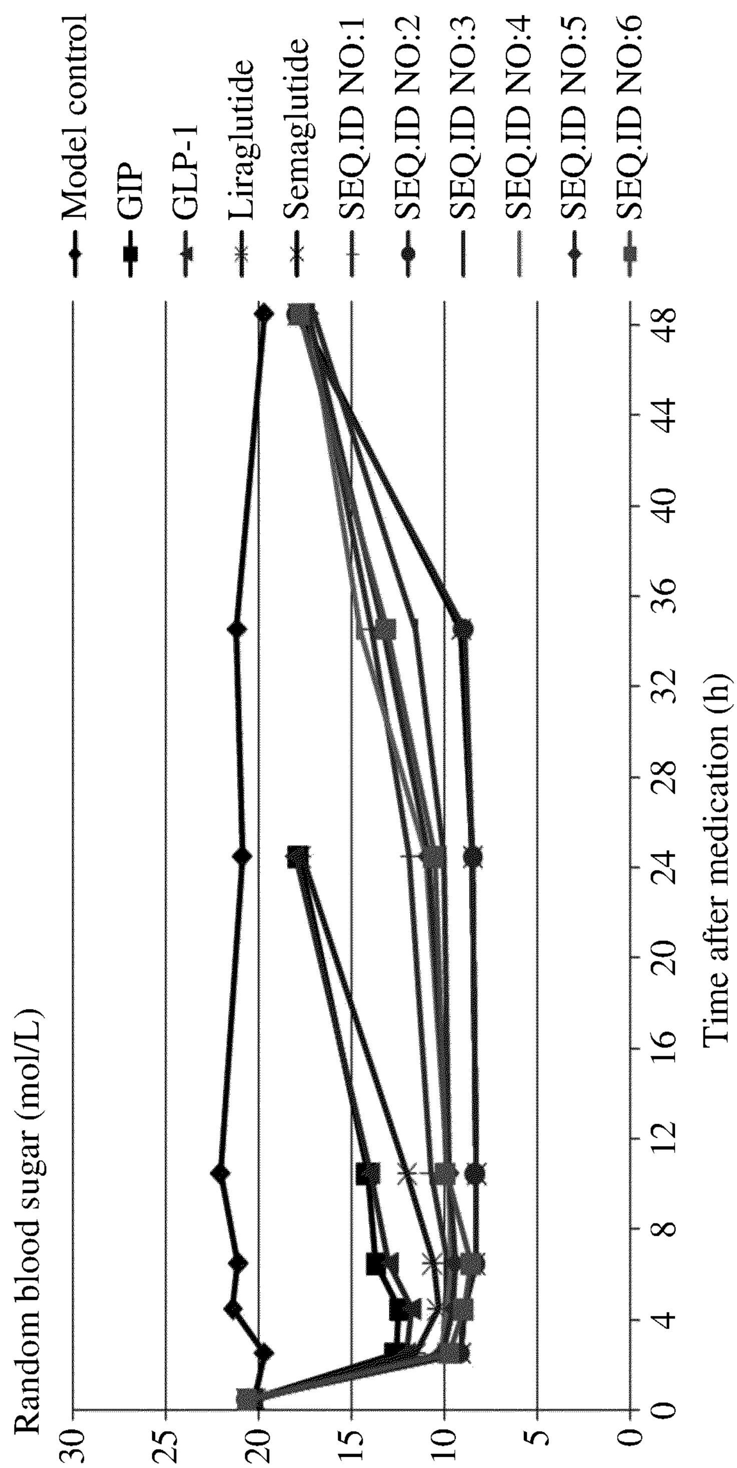
Random blood sugar (mol/L)
30
25
20
15
10
5
0
0
4
8
12
16
20
24
28
32
36
40
44
48
Time after medication (h)
Model control
GIP
GLP-1
Liraglutide
Semaglutide
SEQ.ID NO:1
SEQ.ID NO:2
SEQ.ID NO:3
SEQ.ID NO:4
SEQ.ID NO:5
SEQ.ID NO:6

**FIG. 1**

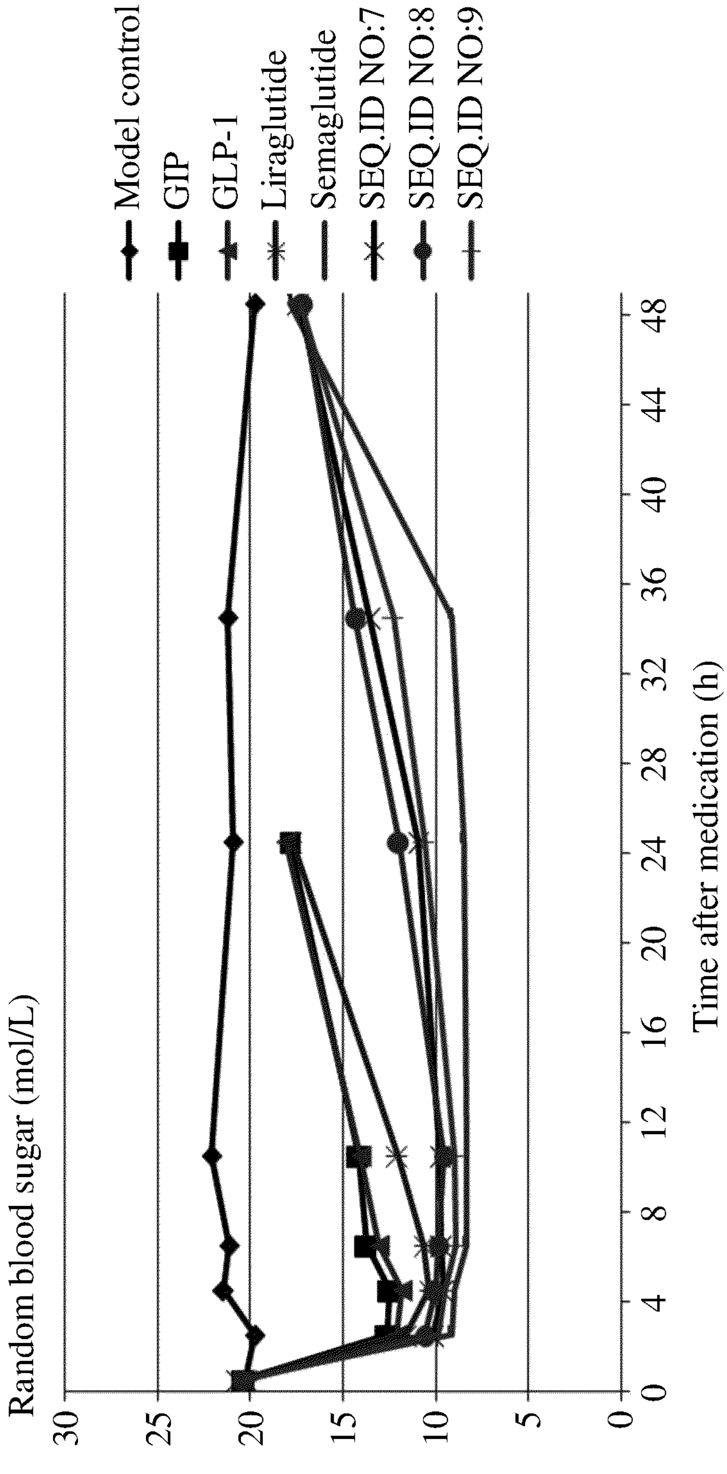
Random blood sugar (mol/L)
30
25
20
15
10
5
0
0
4
8
12
16
20
24
28
32
36
40
44
48
Time after medication (h)
Model control
GIP
GLP-1
Liraglutide
Semaglutide
SEQ.ID NO:7
SEQ.ID NO:8
SEQ.ID NO:9

FIG. 2

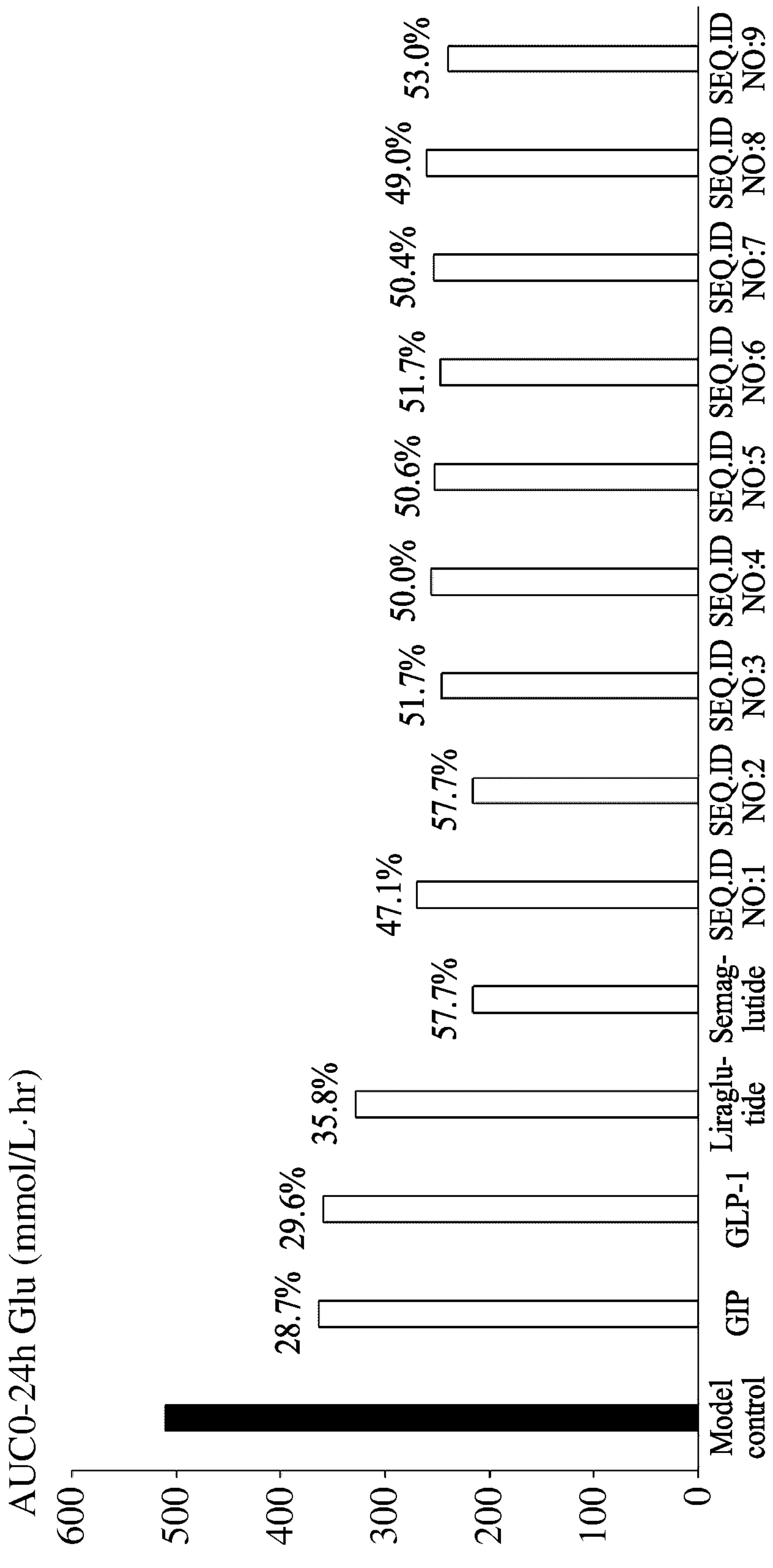
AUC0-24h Glu (mmol/L·hr)
600
500
400
300
200
100
0
28.7%
29.6%
35.8%
57.7%
47.1%
57.7%
51.7%
50.0%
50.6%
51.7%
50.4%
49.0%
53.0%
Model control
GIP
GLP-1
Liraglu-tide
Semag-lutide
SEQ.ID NO:1
SEQ.ID NO:2
SEQ.ID NO:3
SEQ.ID NO:4
SEQ.ID NO:5
SEQ.ID NO:6
SEQ.ID NO:7
SEQ.ID NO:8
SEQ.ID NO:9

FIG. 3

**INTERNATIONAL SEARCH REPORT**

International application No. **PCT/CN2019/110848**

**A. CLASSIFICATION OF SUBJECT MATTER**

C07K 14/00(2006.01)i; C07K 1/06(2006.01)i; C07K 1/04(2006.01)i; A61K 38/16(2006.01)i; A61P 3/04(2006.01)i; A61P 3/10(2006.01)i

According to International Patent Classification (IPC) or to both national classification and IPC

**B. FIELDS SEARCHED**

Minimum documentation searched (classification system followed by classification symbols)

C07K; A61K; A61P

Documentation searched other than minimum documentation to the extent that such documents are included in the fields searched

Electronic data base consulted during the international search (name of data base and, where practicable, search terms used)

VEN, CNABS, PubMed, ISI Web of Knowledge, NCBI Genbank, EMBL-EBI, CNKI: applicant/inventor, GIP, GLP-1, agonist, dual, diabetes, fat, 双激动, 葡萄糖依赖性促胰岛素多肽, 胰高血糖素样肽-1, 激动剂, 糖尿病, 肥胖

**C. DOCUMENTS CONSIDERED TO BE RELEVANT**

| Category* | Citation of document, with indication, where appropriate, of the relevant passages | Relevant to claim No. |
|---|---|---|
| X | CN 107207576 A (ELI LILLY AND COMPANY) 26 September 2017 (2017-09-26) claims 13-18, description paragraphs 65-73, 84, 225-242, embodiments 1-3, sequences 3 and 5 | 1-10 |
| X | CN 108348583 A (ELI LILLY AND COMPANY) 31 July 2018 (2018-07-31) abstract, sequences 13-15, description paragraphs 388-411, claims 1-4 | 1-10 |
| X | CN 108135981 A (ELI LILLY AND COMPANY) 08 June 2018 (2018-06-08) abstract, sequences 13-15, description paragraphs 179-180, claims 1-20 | 1-10 |
| A | WO 2011119657 A1 (ELI LILLY AND COMPANY et al.) 29 September 2011 (2011-09-29) entire document | 1-10 |
| A | JUAN, P.F. et al. "The Sustained Effects of a Dual GIP/GLP-1 Receptor Agonist, NNC0090-2746, in Patients With Type 2 Diabetes" *CELL METABOLISM,* Vol. 26, 01 August 2017 (2017-08-01), pages 343-352, e1-e2 | 1-10 |
| A | COSKUN, T. et al. "LY3298176, a novel dual GIP and GLP-1 receptor agonist for the treatment of type 2 diabetes mellitus: From Discovery to Clinical Proof of Concept" *MOLECULAR METABOLISM,* Vol. 18, 03 October 2018 (2018-10-03), pp. 3-14 | 1-10 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **21 June 2020** | **09 July 2020** |

| Name and mailing address of the ISA/CN | Authorized officer |
|---|---|
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No. **PCT/CN2019/110848**

**C. DOCUMENTS CONSIDERED TO BE RELEVANT**

| Category* | Citation of document, with indication, where appropriate, of the relevant passages | Relevant to claim No. |
|---|---|---|
| A | JUAN, P.F. et al. "Efficacy and Safety of LY3298176, a Novel Dual GIP and GLP-1 Receptor Agonist, in Patients With Type 2 Diabetes: A Randomised, Placebo-Controlled and Active Comparator-Controlled Phase 2 Trial" *LANCET,* Vol. 392, 04 October 2018 (2018-10-04), pp. 2180-2193 | 1-10 |
| A | 杨菊 等 (YANG, Ju et al.). "双受体激动剂CI-1206拮抗Aβ1-42所致小鼠空间学习记忆损伤的作用 (Dual co-agonist CI-1206 antagonizes Aβ1-42-induced impairments in spatial learning and memory in mice)" *中国应用生理学杂志 (Chinese Journal of Applied Physiology),* Vol. 32, No. 6, 31 December 2016 (2016-12-31), pp. 567-570 | 1-10 |

Form PCT/ISA/210 (second sheet) (January 2015)

**INTERNATIONAL SEARCH REPORT**

International application No.
**PCT/CN2019/110848**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

   [1] Upon verification, the priority claim is established.

Form PCT/ISA/210 (continuation of first sheet) (January 2015)

**INTERNATIONAL SEARCH REPORT**
**Information on patent family members**

International application No.
**PCT/CN2019/110848**

| Patent document cited in search report | Publication date (day/month/year) | Patent family member(s) | Publication date (day/month/year) |
|---|---|---|---|
| CN 107207576 A | 26 September 2017 | MX 2017008927 A | 11 October 2017 |
| | | BR 112017010596 A2 | 06 March 2018 |
| | | WO 2016111971 A1 | 14 July 2016 |
| | | US 9474780 B2 | 25 October 2016 |
| | | CA 2973352 A1 | 14 July 2016 |
| | | EA 201791281 A1 | 30 November 2017 |
| | | PT 3242887 T | 29 October 2019 |
| | | AU 2016205435 B2 | 29 March 2018 |
| | | DO P2017000153 A | 15 July 2017 |
| | | PE 20170954 A1 | 13 July 2017 |
| | | AR 103242 A1 | 26 April 2017 |
| | | CL 2017001760 A1 | 16 March 2018 |
| | | PL 3242887 T3 | 28 February 2020 |
| | | SV 2017005453 A | 27 August 2018 |
| | | PE 09542017 A1 | 13 July 2017 |
| | | ES 2747928 T3 | 12 March 2020 |
| | | SG 11201705603 Y A | 30 August 2017 |
| | | CA 2973352 C | 26 March 2019 |
| | | JP 2017507124 A | 16 March 2017 |
| | | KR 20170092661 A | 11 August 2017 |
| | | HU E045860 T2 | 28 January 2020 |
| | | EP 3242887 B1 | 14 August 2019 |
| | | TN 2017000198 A1 | 19 October 2018 |
| | | PH 12017501252 A1 | 30 October 2017 |
| | | LT 3242887 T | 11 November 2019 |
| | | DK 3242887 T3 | 02 September 2019 |
| | | SI 3242887 T1 | 30 October 2019 |
| | | RS 59146 B1 | 30 September 2019 |
| | | EA 201892057 A1 | 28 February 2019 |
| | | KR 20190026967 A | 13 March 2019 |
| | | JP 6219534 B2 | 25 October 2017 |
| | | IL 252499 D0 | 31 July 2017 |
| | | EP 3597662 A1 | 22 January 2020 |
| | | JP 6545766 B2 | 17 July 2019 |
| | | MA 41315 B1 | 29 November 2019 |
| | | EA 031591 B1 | 31 January 2019 |
| | | JP 2018052933 A | 05 April 2018 |
| | | ZA 201703930 B | 26 June 2019 |
| | | AU 2016205435 A1 | 08 June 2017 |
| | | JP 2019203000 A | 28 November 2019 |
| | | TW 201636362 A | 16 October 2016 |
| | | NZ 732000 A | 30 November 2018 |
| | | EP 3242887 A1 | 15 November 2017 |
| | | UA 118239 C2 | 10 December 2018 |
| | | MD 3242887 T2 | 30 November 2019 |
| | | US 2016199438 A1 | 14 July 2016 |
| | | TW I582109 B | 11 May 2017 |
| | | KR 101957620 B1 | 13 March 2019 |
| | | HR P20191614 T1 | 13 December 2019 |
| CN 108348583 A | 31 July 2018 | AU 2016344433 A1 | 19 April 2018 |

Form PCT/ISA/210 (patent family annex) (January 2015)

**INTERNATIONAL SEARCH REPORT**
**Information on patent family members**

International application No.
**PCT/CN2019/110848**

| Patent document cited in search report | Publication date (day/month/year) | Patent family member(s) | Publication date (day/month/year) |
|---|---|---|---|
| | | TW I622596 B | 01 May 2018 |
| | | JP 6321299 B2 | 09 May 2018 |
| | | US 9884093 B2 | 06 February 2018 |
| | | EP 3368061 B1 | 07 August 2019 |
| | | EA 201890676 A1 | 31 January 2019 |
| | | US 2018104312 A1 | 19 April 2018 |
| | | CA 3003242 A1 | 04 May 2017 |
| | | WO 2017074715 A1 | 04 May 2017 |
| | | BR 112018006920 A2 | 06 November 2018 |
| | | IL 258092 D0 | 31 May 2018 |
| | | AU 2016344434 B2 | 06 June 2019 |
| | | WO 2017074714 A1 | 04 May 2017 |
| | | JP 2018508464 A | 29 March 2018 |
| | | TW 201726717 A | 01 August 2017 |
| | | KR 20180053747 A | 23 May 2018 |
| | | AU 2016344433 B2 | 06 December 2018 |
| | | JP 2018135346 A | 30 August 2018 |
| | | JP 6354017 B2 | 11 July 2018 |
| | | AR 106325 A1 | 03 January 2018 |
| | | CN 108135981 A | 08 June 2018 |
| | | ZA 201801756 B | 30 October 2019 |
| | | ZA 201802371 B | 29 January 2020 |
| | | EP 3368060 A1 | 05 September 2018 |
| | | ES 2747908 T3 | 12 March 2020 |
| | | EP 3368061 A1 | 05 September 2018 |
| | | MX 2018005129 A | 06 June 2018 |
| | | MX 2018005132 A | 06 June 2018 |
| | | NZ 740644 A | 25 October 2019 |
| | | US 9764004 B2 | 19 September 2017 |
| | | CA 3000538 A1 | 04 May 2017 |
| | | EA 201890634 A1 | 31 October 2018 |
| | | PL 3368061 T3 | 31 March 2020 |
| | | AR 106318 A1 | 03 January 2018 |
| | | BR 112018007225 A2 | 16 October 2018 |
| | | US 2017114115 A1 | 27 April 2017 |
| | | PT 3368061 T | 28 October 2019 |
| | | AU 2016344434 A1 | 07 June 2018 |
| | | US 2017112904 A1 | 27 April 2017 |
| | | JP 2018504376 A | 15 February 2018 |
| CN 108135981 A | 08 June 2018 | AU 2016344433 A1 | 19 April 2018 |
| | | TW I622596 B | 01 May 2018 |
| | | JP 6321299 B2 | 09 May 2018 |
| | | US 9884093 B2 | 06 February 2018 |
| | | EP 3368061 B1 | 07 August 2019 |
| | | EA 201890676 A1 | 31 January 2019 |
| | | US 2018104312 A1 | 19 April 2018 |
| | | CN 108348583 A | 31 July 2018 |
| | | CA 3003242 A1 | 04 May 2017 |
| | | WO 2017074715 A1 | 04 May 2017 |
| | | BR 112018006920 A2 | 06 November 2018 |

Form PCT/ISA/210 (patent family annex) (January 2015)

**INTERNATIONAL SEARCH REPORT**
**Information on patent family members**

International application No.
**PCT/CN2019/110848**

| Patent document cited in search report | Publication date (day/month/year) | Patent family member(s) | Publication date (day/month/year) |
|---|---|---|---|
| | | IL 258092 D0 | 31 May 2018 |
| | | AU 2016344434 B2 | 06 June 2019 |
| | | WO 2017074714 A1 | 04 May 2017 |
| | | JP 2018508464 A | 29 March 2018 |
| | | TW 201726717 A | 01 August 2017 |
| | | KR 20180053747 A | 23 May 2018 |
| | | AU 2016344433 B2 | 06 December 2018 |
| | | JP 2018135346 A | 30 August 2018 |
| | | JP 6354017 B2 | 11 July 2018 |
| | | AR 106325 A1 | 03 January 2018 |
| | | ZA 201801756 B | 30 October 2019 |
| | | ZA 201802371 B | 29 January 2020 |
| | | EP 3368060 A1 | 05 September 2018 |
| | | ES 2747908 T3 | 12 March 2020 |
| | | EP 3368061 A1 | 05 September 2018 |
| | | MX 2018005129 A | 06 June 2018 |
| | | MX 2018005132 A | 06 June 2018 |
| | | NZ 740644 A | 25 October 2019 |
| | | US 9764004 B2 | 19 September 2017 |
| | | CA 3000538 A1 | 04 May 2017 |
| | | EA 201890634 A1 | 31 October 2018 |
| | | PL 3368061 T3 | 31 March 2020 |
| | | AR 106318 A1 | 03 January 2018 |
| | | BR 112018007225 A2 | 16 October 2018 |
| | | US 2017114115 A1 | 27 April 2017 |
| | | PT 3368061 T | 28 October 2019 |
| | | AU 2016344434 A1 | 07 June 2018 |
| | | US 2017112904 A1 | 27 April 2017 |
| | | JP 2018504376 A | 15 February 2018 |
| WO 2011119657 A1 | 29 September 2011 | KR 101407775 B1 | 17 June 2014 |
| | | JP 2013523647 A | 17 June 2013 |
| | | AU 2011232597 A1 | 30 August 2012 |
| | | EC SP12012181 A | 30 November 2012 |
| | | ZA 201206832 B | 26 March 2014 |
| | | AR 080592 A1 | 18 April 2012 |
| | | MX 2012011127 A | 15 October 2012 |
| | | MA 34077 B1 | 05 March 2013 |
| | | CN 102821779 A | 12 December 2012 |
| | | CN 102821779 B | 11 March 2015 |
| | | EA 022489 B1 | 29 January 2016 |
| | | ES 2568796 T3 | 04 May 2016 |
| | | EA 201270723 A1 | 30 January 2013 |
| | | CR 20120457 A | 16 October 2012 |
| | | PE 20130523 A1 | 25 April 2013 |
| | | TW 201201829 A | 16 January 2012 |
| | | BR 112012024373 A2 | 10 January 2017 |
| | | DO P2012000244 A | 15 February 2013 |
| | | US 8815811 B2 | 26 August 2014 |
| | | SG 184289 A1 | 29 November 2012 |
| | | CL 2012002635 A1 | 21 December 2012 |

Form PCT/ISA/210 (patent family annex) (January 2015)

**INTERNATIONAL SEARCH REPORT**
**Information on patent family members**

International application No.
**PCT/CN2019/110848**

| Patent document cited in search report | Publication date (day/month/year) | Patent family member(s) | | | Publication date (day/month/year) |
|---|---|---|---|---|---|
| | | EP | 2552471 | B1 | 16 March 2016 |
| | | CO | 6630128 | A2 | 01 March 2013 |
| | | PE | 05232013 | A1 | 25 April 2013 |
| | | TW | I535450 | B | 01 June 2016 |
| | | AU | 2011232597 | B2 | 29 January 2015 |
| | | TN | 2012000432 | A1 | 30 January 2014 |
| | | US | 2011237503 | A1 | 29 September 2011 |
| | | CA | 2794664 | A1 | 29 September 2011 |
| | | KR | 20120133402 | A | 10 December 2012 |
| | | GT | 201200263 | A | 05 December 2013 |
| | | EP | 2552471 | A1 | 06 February 2013 |
| | | JP | 5887335 | B2 | 16 March 2016 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013164483 A
- WO 2014192284 A
- WO 2011119657 A